# EUROPÄISCHE PATENTSCHRIFT

(11) **EP 2 282 797 B1**
(45) Veröffentlichungstag und Bekanntmachung des Hinweises auf die Patenterteilung: **08.05.2019**
(21) Anmeldenummer: 09723966.9
(22) Anmeldetag: 24.03.2009
(51) Int. Cl.: A61B 5/0205, A61B 5/021, A61B 5/0496, A61B 5/08, A61B 5/16, A61B 5/00, A61M 21/00, A61B 7/00, A61M 21/02

(54) **SYSTEM UND VERFAHREN ZUR ENTSPANNUNG SOWIE ZUR UNTERSTÜTZUNG UND ÜBERWACHUNG EINES KURZSCHLAFES**
SYSTEM AND METHOD FOR RELAXATION AND FOR SUPPORTING AND MONITORING A NAP
SYSTÈME ET PROCÉDÉ POUR SE DÉTENDRE AINSI QUE POUR FAVORISER ET SURVEILLER UNE SIESTE

(30) Priorität: 28.03.2008 DE 102008016768
(43) Veröffentlichungstag der Anmeldung: 16.02.2011
(73) Patentinhaber: Schultheiss, Carolin, 80796 München (DE); Schultheiss, Georg, 71292 Friolzheim (DE)
(72) Erfinder: Schultheiss, Carolin, 80796 München (DE); Schultheiss, Georg, 71292 Friolzheim (DE)
(74) Vertreter: Hoeger, Stellrecht & Partner Patentanwälte mbB
(86) Internationale Anmeldenummer: PCT/EP2009/053466
(87) Internationale Veröffentlichungsnummer: WO 2009/118319

(56) Entgegenhaltungen:
- EP-A- 0 495 988
- WO-A-2005/000385
- DE-A1- 19 549 297
- DE-A1- 19 642 316
- US-A- 5 954 629

## Beschreibung

Die vorliegende Erfindung betrifft ein System zur Unterstützung, Einleitung und Überwachung eines Kurzschlafes eines Menschen, mit einer Abschirmeinrichtung zum mindestens teilweisen Abschirmen des Menschen von seiner Umgebung und mit einer Entspannungseinrichtung zum Einleiten des Einschlafens des Menschen, wobei es ausgebildet ist zur Überwachung eines Kurzschlafs eines Menschen und eine Schlafdetektionseinrichtung (16) umfasst zum Feststellen, ob der Mensch (11) eingeschlafen ist.

Des Weiteren betrifft die vorliegende Erfindung ein Verfahren zur Unterstützung, Einleitung und Überwachung eines Kurzschlafes eines Menschen, bei welchem der Mensch mindestens teilweise von seiner Umgebung abgeschirmt und eine Entspannung und ein Einleiten des Einschlafens des Menschen durchgeführt wird.

Schlafgewohnheiten, auch als Schlafkulturen bezeichnet, der Menschen in unterschiedlichen Kulturregionen unterscheiden sich deutlich. Bekannt sind drei Grundmuster für Schlafgewohnheiten. In der westlichen Welt, vorwiegend in Europa und Nordamerika, herrscht der nächtliche Monophasenschlaf vor, bei dem die Menschen nachts etwa sieben bis acht Stunden schlafen. Es handelt sich dabei um den längsten Nachtschlaf bei den drei Grundmustern. Die südländische Siestakultur hingegen kennt einen etwas kürzeren Nachtschlaf und zusätzlich eine längere Tagschlafphase. Und schließlich ist die "Nickerchenkultur" mit kurzem Nachtschlaf und mehreren, über den Tag verteilten, kurzen, sogenannten Power-Schläfchen bekannt. Diese Art des Schlafens wird in Afrika und im fernen Osten, insbesondere in Indien, China und vor allem in Japan, praktiziert. Je höher der gesellschaftliche Rang einer Person ist, umso demonstrativer ist sein kurzer Power-Schlaf, auch im Büro. In Japan ist diese Schlafgewohnheit als "Inemuri" bekannt und wird bereits in der Grundschule eingeübt.

Mehrere Kurzschläfchen auch tagsüber erhöhen die Leistungsfähigkeit eines Menschen.

Aus der US 5,954,629 ist ein Hirnwellenerzeugungssystem bekannt. In der WO 2005/000385 A1 sind ein Verfahren und ein System zum Überwachen und Steuern von Schlafzyklen offenbart. Eine Vorrichtung und ein Verfahren zum Wecken einer schlafenden Person ist in der DE 196 42 316 A1 beschrieben. Aus der DE 195 49 297 A1 sind ein Verfahren und eine Vorrichtung zur Beeinflussung der menschlichen Psyche bekannt. Und schließlich offenbart die EP 0 495 988 A1 ein Hilfsinstrument zum Einleiten eines Schlafes oder dergleichen durch Einsatz von Licht.

Es ist daher Aufgabe der vorliegenden Erfindung, ein System und ein Verfahren der eingangs beschriebenen Art so zu verbessern, dass auch ungeübten Menschen, welche den Kurzschlaf weder geübt noch trainiert haben, ein erholsamer Kurzschlaf ermöglicht wird.

Diese Aufgabe wird bei einem System der eingangs beschriebenen Art erfindungsgemäß dadurch gelöst, dass es eine Weckeinrichtung zum Wecken des Menschen vor Eintritt der Tiefschlafphase umfasst.

Das erfindungsgemäß ausgebildete System ermöglicht es einem Menschen insbesondere beim Einschlafen zu helfen und zu überwachen, wie lange der Mensch schläft. Der Kurzschlaf, wenn er eine bestimmte Dauer übersteigt, ist nicht mehr erholsam, da nicht ausgeschlossen werden kann, dass der Mensch während einer Tiefschlafphase aufwacht, wodurch das Erwachen beliebig erschwert wird. Das System ermöglicht daher, sicherzustellen, dass der Mensch nicht zu lange schläft. Mit der Schlafdetektionseinrichtung kann insbesondere ein Schlafzustand detektiert werden, und zwar am Einfachsten derart, ob der Mensch schläft oder nicht. Sobald der Mensch eingeschlafen ist und dies detektiert wird, beginnt die eigentliche Schlafdauer. Der Schlaf wird mittels der Weckeinrichtung nach Ablauf der vorgegebenen Schlafdauer oder gegebenenfalls vor Ablauf der vorgegebenen Zeit, vorzugsweise während einer REM-Phase, beendet, sodass der Mensch optimal ausgeruht ist. Schlafdauern können beliebig in einem Bereich von beispielsweise 5 Minuten bis maximal 60 Minuten vorgegeben werden. Nach einem solchen Kurzschlaf ist der Mensch fit und wach, ohne die Einnahme anregender Stoffe, wie beispielsweise Koffein, durch Konsum von Kaffee oder anderen koffeinhaltigen Getränken. Es ist auch denkbar, das System beispielsweise in öffentlichen Verkehrsmitteln einzusetzen. Mit der Weckeinrichtung kann sichergestellt werden, dass zum Beispiel Haltestellen nicht "verschlafen" werden. Des Weiteren stellt das System auch sicher, dass Mittagspausen eingehalten und nicht überschritten werden. Das System führt bei dem es nutzenden Menschen zu einem Stressabbau und ferner zu einer Minimierung von Arbeits- und Verkehrsunfällen infolge erhöhter Konzentrations- und Reaktionsfähigkeitsdefizite aufgrund Übermüdung. Die Aktivität und die Leistungsfähigkeit des Menschen wird erhöht, ebenso werden das Wohlbefinden und die Lebensfreude gesteigert. Ferner eignet sich das System, um auch einen Kurzschlaf im Beisein von anderen Menschen zu erleichtern, was vorzugsweise mithilfe der Abschirmeinrichtung erreicht wird, die beispielsweise einen Sicht- und/oder Lärmschutz, quasi eine Art "Kokon" bildet, wodurch ein Maximum an Privatsphäre auch in einem öffentlichen Umfeld erreicht wird. Das System ist prinzipiell an jedem Ort einsetzbar. Es ist auch nur zur Entspannung geeignet, das heißt es ist nicht zwingend erforderlich, das System zu einem Kurzschlaf zu nutzen. Es ist bekannt, dass auch eine entsprechende Ruhephase bei beispielsweise geschlossenen Augen und der Vorstellung, sich in einem weißen, völlig geräuschlosen Raum bei warmen Licht zu befinden, entsprechend einer Übung autogenen Trainings, für manche Menschen so effektvoll ist, wie ein echter Kurzschlaf. Auch hier ermöglicht es das System, Ungeübten zur Entspannung zu verhelfen.

Um den Menschen oder Nutzer beziehungsweise Anweder des Systems insbesondere in einem öffentlichen, von akustischen und/oder optischen Reizen überfluteten Umfeld abzuschirmen, ist es günstig, wenn die Abschirmeinrichtung eine akustische und/oder optische Abschirmeinrichtung umfasst.

Besonders effizient und klein kann die Abschirmeinrichtung ausgebildet werden, wenn die optische Abschirmeinrichtung eine Gesichts- und/oder Augenabdeckungseinrichtung umfasst zum mindestens teilweisen Bedecken oder Abdecken des Gesichts und/oder mindestens eines Auges oder beider Augen des Menschen. Insbesondere kann sie auch derart ausgebildet sein, dass Mund und/oder Nase abgedeckt werden. So ist es auf einfache Weise möglich, den Menschen von optischen Reizen abzuschirmen.

Eine besonders gute Abschirmung kann erreicht werden, wenn die optische Abschirmeinrichtung in Richtung auf das Gesicht des Menschen hin lichtundurchlässig ist.

Vorzugsweise ist die optische Abschirmeinrichtung in Richtung vom Gesicht des Menschen weg mindestens teilweise lichtdurchlässig. Dies hat den Vorteil, dass der Mensch insbesondere beim Erwachen seine Umgebung wahrnehmen kann und nicht unfallgefährdet ist.

Vorzugsweise ist eine Lichtdurchlässigkeit der optischen Abschirmeinrichtung veränderbar. Dies gestattet es, beispielsweise auch zum Aufwachen die Lichtdurchlässigkeit zu erhöhen und den Menschen quasi sukzessive wieder in den "Alltag" zurückzuführen.

Gemäß einer weiteren bevorzugten Ausführungsform des Systems kann vorgesehen sein, dass die optische Abschirmeinrichtung von einer Transportstellung, in welcher sie eine minimale Fläche überdeckt, in eine Betriebsstellung, in welcher die Augen des Menschen mindestens teilweise bedeckt oder abgedeckt sind, bringbar ist und/oder umgekehrt. Eine derartige Ausbildung der optischen Abschirmeinrichtung ermöglicht es, sie auf einfache Weise zu transportieren. Sie kann so beispielsweise klein zusammengepackt werden. Selbstverständlich kann auch die akustische Abschirmeinrichtung von einer Transportstellung in eine Betriebsstellung bringbar sein, um den Transport des Systems insgesamt zu erleichtern.

Damit nur diejenigen Körperpartien des Menschen abgeschirmt werden, die tatsächlich abgeschirmt werden müssen, ist es vorteilhaft, wenn die optische Abschirmeinrichtung mindestens teilweise lichtundurchlässig ist.

Besonders einfach wird der Aufbau des Systems, wenn die Gesichts- und/oder Augenabdeckungseinrichtung mindestens eine Augenklappe und/oder ein Visier und/oder eine Mundabdeckung umfasst.

In eine besonders kleine und kompakte Form, insbesondere für Transportzwecke, lässt sich das System bringen, wenn die Gesichts- und/oder Augenabdeckungseinrichtung mehrteilig ausgebildet ist und wenn mindestens zwei Teile der Gesicht- und/oder Augenabdeckungseinrichtung relativ zueinander bewegbar angeordnet sind. Beispielsweise können sie um eine Schwenkachse relativ zueinander verschwenkbar angeordnet sein, vorzugsweise zur Ausbildung einer Visiereinrichtung.

Günstigerweise umfasst die akustische Abschirmeinrichtung eine aktive und/oder passive Lärm- und/oder Gehörschutzeinrichtung. Mit einer solchen akustischen Abschirmeinrichtung ist es möglich, Lärm und Geräusche, seien es temporär oder dauerhaft auftretende Störungen, gezielt vom Menschen fern zu halten, um sowohl das Einschlafen als auch das Schlafen zu erleichtern und zu verbessern.

Besonders einfach wird der Aufbau des Systems, wenn die passive Lärm- und/oder Gehörschutzeinrichtung in Form eines Kapselgehörschutzes oder in Form von Gehörschutzstöpseln ausgebildet ist. Ein Kapselgehörschutz kann insbesondere in Form eines Aufsatzes für die Ohren ausgebildet sein, welcher die Ohrmuschel vollständig umschließt, also kapselt. Er kann auch durch einen Kopfhörer in geschlossener Bauform realisiert sein.

Vorteilhafterweise umfasst die aktive Lärm- und/oder Gehörschutzeinrichtung mindestens einen, vorzugsweise zwei, Ohrhörer mit einer aktiven Umgebungsgeräuschminderungseinrichtung zur aktiven Reduzierung von permanent und/oder temporär auftretenden Umgebungsgeräuschen. Insbesondere kann ein sogenannter "Noise-cancelling"-Ohr- oder Kopfhörer einen Teil des Systems bilden, mit dem Lärm- oder Geräuschquellen mindestens teilweise, vorzugsweise ganz, ausgeschaltet werden können. Eine aktive Lärm- und/oder Gehörschutzeinrichtung zeichnet sich insbesondere dadurch aus, dass Lärm oder Geräusche detektiert und aktiv durch Überlagerung entsprechender gegenphasiger Geräusche mindestens teilweise eliminiert werden unter Anwendung des physikalischen Prinzips der Superposition.

Damit das System durch den Mensch einfach und sicher angewendet werden kann, ist es vorteilhaft, wenn es einen Träger umfasst, an welchem die Abschirmeinrichtung und/oder die Entspannungseinrichtung und/oder die Schlafdetektionseinrichtung und/oder die Weckeinrichtung mindestens teilweise gehalten sind. Je weniger Einrichtungen, die in unmittelbarer Nähe insbesondere des Kopfes der Person erforderlich sind, am Träger angeordnet und gehalten sind, umso leichter wird eine durch den Träger und die an diesem angeordneten und gehaltenen Einrichtungen ausgebildete Trägereinheit. Dies erhöht den Tragekomfort und erleichtert das Entspannen und gegebenenfalls Einschlafen des Menschen.

Besonders einfach und sicher lässt sich der Träger am Kopf eines Menschen tragen, wenn er ein Kopf- und/oder ein Nase- und/oder ein Ohrenauflageteil umfasst zum An- und/oder Auflegen des Trägers auf den Kopf und/oder die Nase und/oder mindestens ein Ohr, vorzugsweise zwei Ohren, des Menschen. Selbstverständlich können auch zwei Ohrenauflageteile vorgesehen werden.

Vorteilhafterweise ist der Träger in Form eines Helmes, einer Mütze, eines Hutes, eines Kopftuches, einer Haube, eines Schutzschildes oder eines Schals ausgebildet. Derartige Trägerformen eignen sich hervorragend, um von einem Menschen am Kopf getragen zu werden.

Selbstverständlich kann es auch günstig sein, wenn der Träger brillenförmig oder in Form einer Brille ausgebildet ist. Er lässt sich dann wie eine Brille tragen.

Das System muss nicht zwingend in Form eines persönlichen Systems ausgebildet sein, dass heißt in Form eines Systems, das nur von einer Person genutzt wird. Es wäre auch denkbar, das System beispielsweise öffentlich zugänglich zu machen, insbesondere in öffentlichen Verkehrsmitteln. Um bei der Benutzung eines solchen, optional fest installierten Systems die Gefahr der Übertragung von Krankheiten von einem ersten Benutzer auf einen zweiten Benutzer des Systems zu minimieren oder ganz auszuschließen, ist es günstig, wenn eine Desinfektionseinrichtung zum Desinfizieren mindestens einer Einrichtung des Systems vorgesehen ist. Die Desinfektionseinrichtung kann in das System integriert sein, beispielsweise in den Träger in Form eines Helms, welcher durch Austreten und Verteilen eines Desinfektionsmittels, zum Beispiel in Form eines Gases oder einer Flüssigkeit, die geeignet sind, beispielsweise den Helm zu desinfizieren, für die unbedenkliche Nutzung eines nächsten Benutzers vorbereitet werden kann.

Damit die Desinfektionseinrichtung nicht unnötigerweise zum Einsatz kommt, umfasst das System vorteilhafterweise eine Desinfektionszustanddetektionseinrichtung zum Detektieren eines Desinfektionszustands des Systems oder mindestens einer Einrichtung desselben. Wenn detektiert wird, dass eine Person das System benutzt und es nach Benutzung freigegeben hat, dann kann insbesondere ein Signal erzeugt werden, welches einem Desinfektionszustand entspricht, gemäß dem das System zu Desinfizieren ist.

Um den Benutzer über den Desinfektionszustand eines öffentlichen Systems zu informieren, umfasst das System vorteilhafterweise eine Desinfektionszustandsanzeige zum Anzeigen des detektierten Desinfektionszustands.

Gemäß einer bevorzugten Ausführungsform des Systems ist die Entspannungseinrichtung in Form einer optischen und/oder akustischen und/oder olfaktorischen Entspannungseinrichtung ausgebildet. Mit einer solchen Entspannungseinrichtung kann alternativ oder kombiniert auf die menschlichen Sinne Sehen, Hören und Riechen eingewirkt werden, um gezielt eine Entspannung des Menschen und gegebenenfalls das Einschlafen desselben zu bewirken.

Günstigerweise umfasst die optische Entspannungseinrichtung mindestens eine optische Wiedergabeeinrichtung zum Wiedergeben und Anzeigen von Bildern, Zeichen, Farben und/oder Filmen.

Der Aufbau der optischen Wiedergabeeinrichtung wird besonders einfach, wenn diese mindestens einen Bildschirm, vorzugsweise zwei oder drei, und/oder mindestens ein Leuchtelement umfasst. Mit dem Bildschirm können beliebige Zeichen und Bilder wiedergegeben werden. Das mindestens eine Leuchtelement eignet sich hervorragend, um einen bestimmten Farbeindruck zu erzeugen, um so optisch eine angenehme Atmosphäre für den Nutzer des Systems zu schaffen. Selbstverständlich können auch mehrere Leuchtelemente vorgesehen sein, die optional ihre Farbe zeit- und schlafzustandsabhängig ändern können.

Besonders kostengünstig lässt sich die optische Wiedergabeeinrichtung herstellen, wenn das mindestens eine Leuchtelement eine Leuchtdiode ist. Es kann sich dabei um eine Mono- oder Multicolorleuchtdiode handeln.

Vorzugsweise umfasst die akustische Entspannungseinrichtung mindestens eine akustische Wiedergabeeinrichtung zum Wiedergeben von Musik, Geräuschen oder Tönen und Tonfolgen. Dies gestattet es, den Nutzer des Systems in gewünschter Weise zu beschallen, um ihn zum Entspannen und/oder zum Einschlafen zu bringen.

Der Aufbau des Systems wird besonders einfach, wenn die akustische Wiedergabeeinrichtung mindestens einen Lautsprecher oder mindestens einen Kopf- oder Ohrhörer umfasst. Der Kopf- oder Ohrhörer kann von einem Nutzer direkt am Kopf getragen werden und beispielsweise an einem Träger befestigt sein.

Um beliebige Wiedergabequellen mit dem System koppeln zu können, ist es günstig, wenn mindestens eine Schnittstelle zum Verbinden der Entspannungseinrichtung und/oder Weckeinrichtung mit einer Abspieleinrichtung zum Abspielen von Ton- und/oder Bilddaten vorgesehen ist. Die Schnittstelle, die insbesondere als USB-Schnittstelle ausgebildet sein kann, ermöglicht es, handelsübliche Abspiel- oder Ton- und Bildwiedergabegeräte mit einer oder mehreren Einrichtungen des Systems zu verbinden. Denkbar wären hier insbesondere tragbare CD- und/oder DVD-Abspielgeräte oder sogenannte MP3-Player, MP4-Player, Ton- und Video- oder sonstige Wiedergabesysteme sowie zukünftige neue Varianten oder Neuentwicklungen solcher Geräte.

Optional kann die Abspieleinrichtung zum Abspielen von Ton- und/oder Bilddaten selbstverständlich auch einen Teil des Systems bilden. Insbesondere lassen sich kleine leichte Flashspeicher-Abspielgeräte an einem Träger anordnen und in das System integrieren.

Vorteilhafterweise ist die Abspieleinrichtung zum Abspielen von Musik, Geräuschen und Tönen und Tonfolgen und/oder Filmen, Bildern und/oder Zeichen ausgebildet. Damit kann der Nutzer des Systems gezielt und in gewünschter Weise akustischen und/oder optischen Reizen ausgesetzt werden.

Günstig ist es, wenn das System mindestens eine Speichereinrichtung zum Speichern von Ton- und/oder Bilddaten und/oder Computerprogrammen umfasst. Die Speichereinrichtung ermöglicht es, wahlweise individuell für den Nutzer zusammengestellte Ton- und/oder Bilddaten abzuspeichern und, wenn die Speichereinrichtung mit einer Wiedergabeeinrichtung des Systems verbunden ist, diese Daten entsprechend wiederzugeben. Ferner kann auch ein Computer- und/oder Softwareprogramm, welches in der Speichereinrichtung, beispielsweise einem Datenspeicher, gespeichert ist, auf einer Datenverarbeitungseinrichtung des Systems ablaufen, um die Einrichtungen des Systems entsprechend zu steuern und/oder zu regeln.

Um wahlweise Ton- und/oder Bilddaten dem System zuführen zu können, ist es günstig, wenn es mindestens eine Datenträgerleseeinheit zum Lesen von Ton- und/oder Bilddaten von Datenträgern aufweist. Insbesondere kann die Datenträgerleseeinheit über eine Schnittstelle oder einen geeigneten Datenempfänger, beispielsweise eine USB- oder Firewire-Schnittstelle oder dafür geeignete Bluetootheinrichtungen, verbunden werden.

Besonders einfach wird der Aufbau des Systems sowie kompatibel zu üblichen Speichermedien, wenn die Datenträgerleseeinheit einen Chipkartenleser, einen Speicherkartenleser, ein CD-Laufwerk, ein DVD-Laufwerk, ein Video- und/oder Audiokassettenlaufwerk, ein Tonbandlaufwerk und/oder ein Filmlaufwerk und/oder ein kabelloses Datenempfangssystem und/oder eine Schnittstelle, insbesondere eine USB-Schnittstelle, umfasst.

Um die Entspannung des Nutzers weiter zu steigern, ist es günstig, wenn die olfaktorische Entspannungseinrichtung eine Duftabgabeeinrichtung zum Abgeben von Duftstoffen umfasst. So ist es möglich, mit dem System ein für den Nutzer angenehmes Ambiente zu schaffen und ihm das Entspannen und gegebenenfalls das Einschlafen zu erleichtern.

Damit nur so viel Duftstoffe wie nötig abgegeben werden müssen und um Dritte in der Umgebung des Nutzers nicht zu stören, ist es vorteilhaft, wenn die olfaktorische Entspannungseinrichtung eine Dosiereinrichtung zum Dosieren einer Duftstoffabgabemenge umfasst.

Vorzugsweise umfasst die Schlafdetektionseinrichtung eine Schlafzustandsdetektionseinrichtung zum Detektieren eines Schlaf- und/oder Wachzustands des Menschen. Mit dieser Einrichtung ist es auf einfache Weise möglich, insbesondere die Art des Schlafzustands, das heißt zum Beispiel besondere Schlafphasen, zu detektieren, beispielsweise REM-Schlafphasen oder Tiefschlafphasen, und zu unterscheiden.

Vorzugsweise umfasst die Schlafzustandsdetektionseinrichtung eine optische und/oder akustische Detektionseinrichtung zum Detektieren mindestens einer Vitalfunktion des Menschen. Aus den detektierten Vitalfunktionen kann durch Vergleich mit Daten von Vergleichspersonen, sogenannten Normdaten, ein Schlafzustand des Nutzers des Systems bestimmt werden. Insbesondere ist es vorteilhaft, die Schlafzustandsdetektionseinrichtung einzusetzen, um zu bestimmen, wann der Nutzer eingeschlafen ist, um dann die Weckeinrichtung automatisch nach Ablauf der gewünschten Schlafdauer aktivieren zu können.

Besonders einfach lässt sich die Schlafzustandsdetektionseinrichtung ausbilden, wenn sie eine Atmungsüberwachungseinrichtung umfasst zum Messen und/oder Überwachen einer Atemfrequenz des Menschen. Dabei macht man sich zunutze, dass während des Schlafes eines Menschen die Atemfrequenz signifikant absinkt.

Auf besonders einfache Weise lässt sich die Atemfrequenz des Menschen ermitteln, wenn die Atmungsüberwachungseinrichtung ein Mikrophon umfasst. Mit dem Mikrophon können Atemstöße, die üblicherweise mit Atemgeräuschen verbunden sind, detektiert werden.

Da sich auch der Blutdruck beim Übergang vom Wach- in den Schlafzustand eines Menschen ändert, ist es günstig, wenn die Schlafzustandsdetektionseinrichtung eine Blutdrucküberwachungseinrichtung umfasst zum Messen und/oder Überwachen eines Blutdrucks des Menschen.

Vorzugsweise umfasst die Schlafzustandsdetektionseinrichtung eine Herzfrequenzüberwachungseinrichtung zum Messen und/oder Überwachen einer Herzfrequenz des Menschen. Während des Schlafens sinkt auch die Herzfrequenz signifikant ab, so dass von einer bestimmten Herzfrequenz des Menschen indirekt auf dessen Schlaf- oder Wachzustand geschlossen werden kann.

In bestimmten Schlafzuständen, beispielsweise in REM-Schlafphasen, bewegen sich die Augen des Schlafenden mit hoher Frequenz hin und her. Dieses sogenannte "Rapid Eye Movement" lässt sich besonders einfach überwachen, wenn die Schlafzustandsdetektionseinrichtung eine Augenbewegungsüberwachungseinrichtung umfasst zum Messen und/oder Überwachen einer Augenbewegung. Insbesondere ist es günstig, wenn die Weckeinrichtung während einer REM-Schlafphase aktiviert wird, da ein Mensch aus einer REM-Schlafphase besonders schnell und schonend erwachen kann.

Ferner kann es auch günstig sein, wenn die Schlafzustandsdetektionseinrichtung eine Hirnstromüberwachungseinrichtung umfasst zum Messen und/oder Überwachen von Hirnströmen des Menschen. Auch aus den Hirnströmen kann indirekt auf einen Schlaf- oder Wachzustand des Menschen geschlossen werden.

Gemäß einer bevorzugten Ausführungsform des Systems kann vorgesehen sein, dass die Schlafzustandsdetektionseinrichtung eine Vergleichs- und Zuordnungseinrichtung umfasst zum Vergleichen von mit der Schlafzustandsdetektionseinrichtung gemessenen Istwerten mit Sollwerten von Schlafzuständen und zum Zuordnen eines Ist-Schlafzustands in Abhängigkeit eines Vergleichsergebnisses der Ist- und Sollwerte. Aus den mit den oben beschriebenen Einrichtungen der Schlafzustandsdetektionseinrichtung gemessenen Werten kann so auf einfache Weise und in Echtzeit ein Schlafzustand des Nutzers des Systems durch einfachen Vergleich mit Sollwerten, bei denen es sich insbesondere um für Menschen bekannte Durchschnittswerte handeln kann, ermittelt werden. Denkbar wäre es auch, das System als Selbstlernsystem auszubilden, das sich im Laufe der Zeit auf den jeweiligen Nutzer einstellt, quasi "lernt", welche Schlafgewohnheiten der Nutzer hat.

Um die Schlafdauer des Kurzschlafes vorgeben zu können, ist es vorteilhaft, wenn das System eine Schlafdauervorgabeeinrichtung umfasst.

Hierfür ist es vorteilhaft, wenn die Schlafdauervorgabeeinrichtung eine Eingabeeinheit zum Eingeben einer Schlafdauer aufweist. Damit können gewünschte Schlafdauern individuell eingestellt werden, beispielsweise in Abhängigkeit davon, wie viel überhaupt Zeit für einen Kurzschlaf zur Verfügung steht. Selbstverständlich ist es auch möglich, mit der Eingabeeinheit eine Betriebsdauer des Systems einzugeben, also insbesondere eine Zeit ab einem Start des Systems, die auch als Systemaktivierungszeit bezeichnet werden kann. Die Schlafdauer selbst ist die Zeit, die mit dem Einschlafen des Nutzers beginnt und endet mit der Aktivierung der Weckeinrichtung.

Günstig ist es, wenn die Weckeinrichtung nach Ablauf der vorgegebenen Schlafdauer aktivierbar ist. Vorzugsweise ist sie vor Ablauf der vorgegebenen Schlafdauer nicht aktivierbar. Damit kann vermieden werden, dass der Schlaf vorzeitig unterbrochen wird und der gewünschte Entspannungs- und/oder Erholungseffekt für den Nutzer nicht erreicht wird.

Vorteilhafterweise umfasst die Weckeinrichtung mindestens eine optische und/oder akustische Wiedergabeeinrichtung. Damit lässt sich der Nutzer des Systems optisch und/oder akustisch wecken, beispielsweise durch Beschallung mit Tönen, Musik oder Geräuschen und/oder durch Erzeugen optischer Reize, insbesondere in Form von Bildern, Farben oder Zeichen.

Günstig ist es, wenn die Weckeinrichtung ausgebildet ist zum mindestens teilweisen Entfernen und/oder Abschalten der Abschirmeinrichtung. Beispielsweise kann eine Abdeckungseinrichtung ganz oder teilweise entfernt werden. Optional kann diese auch partiell lichtdurchlässig gemacht werden, um zusätzliche optische Reize auszulösen, die das Aufwecken des Nutzers erleichtern und beschleunigen.

Zum Steuern und/oder Regeln mindestens einer der Einrichtungen des Systems ist es vorteilhaft, wenn mindestens eine Steuer- und/oder Regelungseinrichtung vorgesehen ist. Zusätzlich ist es denkbar, jeder der oben genannten und beschriebenen Einrichtungen des Systems eine eigene Steuer- und/oder Regelungseinrichtung zuzuordnen. Selbstverständlich ist es auch denkbar, nur eine einzige Steuer- und/oder Regelungseinrichtung für zwei oder mehr, insbesondere auch alle Einrichtungen des Systems vorzusehen. Die Steuer- und/oder Regelungseinrichtung kann direkt an einer der Einrichtungen des Systems angeordnet sein oder aber auch von dieser getrennt.

Insbesondere dann, wenn die Steuer- und/oder Regelungseinrichtung getrennt von den Einrichtungen des Systems angeordnet ist, beispielsweise in Form eines MP3-/MP4-Players, zum Beispiel in Form eines "i-pod®" oder Bildtelefons, ist es vorteilhaft, wenn sie mit mindestens einer Einrichtung des Systems mittels einer Kabelverbindung und/oder einer kabellosen Verbindung gekoppelt ist. Insbesondere ist es denkbar, die Steuer- und/oder Regelungseinrichtung als Teil einer Fernbedienung vorzusehen, mit der die einzelnen Einrichtungen des Systems ferngesteuert betätigbar sind. Die Fernbedienung umfasst vorzugsweise auch eine Eingabeeinrichtung, um für die Nutzung des Systems erforderliche Daten einzugeben, beispielsweise die gewünschte Schlafdauer oder die während der Nutzung des Systems gewünschten Bild- und/oder Tondaten. Eine kabellose Verbindung kann insbesondere mittels Funk, Infrarot und/oder Ultraschall realisiert werden.

Günstigerweise ist die kabellose Verbindung eine Bluetoothverbindung. Auf diese Weise können insbesondere alle Bluetooth-fähigen Geräte prinzipiell mit dem System gekoppelt werden. Hierfür eignen sich insbesondere Mobiltelefone, die gleichzeitig auch als Bild- und/oder Tonabspielgeräte eingesetzt werden können.

Grundsätzlich wäre es denkbar, die einzelnen Einrichtungen des Systems zum Zusammenwirken derselben mittels Kabelverbindungen zu verbinden. Um das Systems wahlweise mit anderen Einrichtungen auszustatten oder um beispielsweise einzelne, stationär vorhandene Einrichtungen des Systems, zum Beispiel eine in einem öffentlichen Verkehrsmittel installierte Abschirmeinrichtung, mit anderen Einrichtungen des Systems, die der Nutzer individuell mit sich führt, vorübergehend zur Nutzung und Konfiguration des Systems zu koppeln, ist es günstig, wenn das System eine kabellose Verbindungseinrichtung zum drahtlosen Verbinden einzelner Einrichtungen des Systems untereinander und/oder mit externen Geräten umfasst.

Gemäß einer bevorzugten Ausführungsform der Erfindung kann vorgesehen sein, dass die Steuer- und/oder Regelungseinrichtung mindestens eine Rechen- und/oder Datenverarbeitungseinheit umfasst zum Verrechnen und/oder Verarbeiten von mit einzelnen Einrichtungen des Systems gemessener oder erfasster Daten und/oder vorgegebener Daten oder Sollwerten. Vorzugsweise ist die Rechen- und/oder Datenverarbeitungseinheit im selben Gehäuse untergebracht wie die Steuer- und/oder Regelungseinrichtung. Dadurch kann ein besonders kompakter Aufbau erreicht werden. Insbesondere kann die Steuerung- und/oder Regelungseinrichtung in Form einer Funk-, Infrarot-, Ultraschall- oder Kabelfernbedienung ausgebildet sein, die der Nutzer zum Aktivieren und/oder Bedienen des Systems in der Hand hält oder in einer Tasche eines Kleidungsstücks.

Vorzugsweise ist die Steuer- und/oder Regelungseinrichtung ausgebildet zum Ansteuern der Abschirmeinrichtung und/oder der Entspannungseinrichtung. Insbesondere lassen sich so die Abschirmeinrichtung und/oder die Entspannungseinrichtung in Abhängigkeit des Schlafzustands und der vorgegebenen Schlafdauer in gewünschter Weise beeinflussen.

Vorzugsweise ist die Steuer- und/oder Regelungseinrichtung ausgebildet zum Ansteuern der Duftabgabeeinrichtung. So kann die Art des Dufts, insbesondere dann, wenn mehrere Düfte von der Duftabgabeeinrichtung abgegeben werden können, in Abhängigkeit beispielsweise der Vorlieben des Nutzers, und/oder aber auch deren Dosierung gezielt gesteuert und/oder geregelt werden.

Vorteilhafterweise ist die Steuer- und/oder Regelungseinrichtung mit der Schlafdauervorgabeeinrichtung gekoppelt. So ist es möglich, bei der Steuer- und/oder Regelungseinrichtung nach Ablauf der Schlafdauer die Weckeinrichtung zu aktivieren.

Gemäß einer bevorzugten Ausführungsform des Systems kann vorgesehen sein, dass mit der Steuer- und/oder Regelungseinrichtung die Weckeinrichtung in Abhängigkeit der vorgegebenen Schlafdauer und des mit der Schlafzustandsdetektionseinrichtung detektierten Schlafzustands ansteuerbar ist.

Vorteilhafterweise ist die Weckeinrichtung nur während einer REM-Schlafphase des Menschen aktivierbar. So kann vermieden werden, dass der Nutzer mit der Weckeinrichtung während einer Tiefschlafphase geweckt wird. Eine Ausnahme für die Aktivierung der Weckeinrichtung während einer REM-Schlafphase kann insbesondere dann erfolgen, wenn feste, zeitlich vorgegebene Weckezeiten erforderlich sind, beispielsweise beim Einsatz des Systems in einem öffentlichen Verkehrsmittel oder an einer Haltestelle.

Um eine Austrocknung von Schleimhäuten des Menschen durch Bedeckung von Mund und/oder Nase mit der Abschirmeinrichtung zu vermeiden und um ihn ausreichend mit Frischluft zu versorgen, ist es vorteilhaft, wenn das System eine Belüftungseinrichtung zum Versorgen des Menschen mit Frischluft umfasst. Diese kann insbesondere Belüftungsöffnungen an der Abschirmeinrichtung umfassen, Belüftungskanäle, welche eine Fluidverbindung zwischen einer Umgebung des Nutzers und dem Mund-/Nasenbereich desselben herstellen, oder insbesondere auch mindestens einen kleinen Ventilator, welcher eine Frischluftströmung zum Mund-/Nasenbereich bewirken kann.

Damit das System an beliebigen Orten einsatzfähig ist, ist es vorteilhaft, wenn es eine netzunabhängige Energieversorgungseinheit für mindestens eine der Einrichtungen des Systems umfasst. Vorzugsweise kann mit der netzabhängigen Energieversorgung das gesamte System mit Energie versorgt werden. Die netzunabhängige Energieversorgung kann selbst auch dazu genutzt werden, einzelne Energieversorgungseinheiten des Systems, beispielsweise Akkus von einzelnen Einrichtungen des Systems, nach- und/oder wieder aufzuladen. Selbstverständlich sind auch mehrere netzunabhängige Energieversorgungseinheiten denkbar, optional für jede Einrichtung des Systems eine.

Um das System oder Teile desselben auf einfache Weise transportieren zu können, ist es vorteilhaft, wenn das System eine Transporteinrichtung umfasst zum Aufnehmen des Systems oder mindestens einer Einrichtung desselben für Transportzwecke.

Besonders einfach wird der Aufbau der Transporteinrichtung, wenn sie in Form eines Beutels, einer Tasche oder eines Koffers ausgebildet ist.

Um trotz eines Kurzschlafs für Außenstehende jederzeit erreichbar zu sein, ist es günstig, wenn das System eine Schnittstelle zum Verbinden mit einem Mobiltelefon und/oder dem Internet aufweist. Geht beispielsweise ein Telefonanruf ein, so kann dies signalisiert werden, auch dann, wenn sich der Nutzer gerade in einem Schlafzustand befindet. Der Anruf kann insbesondere auch genutzt werden, um die Weckeinrichtung zu aktivieren.

Vorteilhafterweise umfasst das System eine Eingabeeinrichtung zum Eingeben von Daten und/oder Vorgabewerten für mindestens eine Einrichtung und/oder Vorrichtung des Systems und/oder mit dem System verbundener oder gekoppelter Vorrichtungen oder Geräte. Die Eingabeeinrichtung kann beispielsweise Teil einer Fernbedienung sein. Sie kann auch direkt oder indirekt mit der Steuer- und/oder Regelungseinrichtung des Systems gekoppelt sein.

Die eingangs gestellte Aufgabe wird ferner bei einem Verfahren der eingangs beschriebenen Art erfindungsgemäß dadurch gelöst, dass der Mensch vor Eintritt einer Tiefschlafphase wieder geweckt wird.

Das Verfahren ermöglicht es, in analoger Weise wie das oben beschriebene System, einem Menschen einen Kurzschlaf zu ermöglichen, wobei die Schlafphase vorzugsweise dann beendet wird, wenn sich der Mensch nicht in einer Tiefschlafphase befindet. Im Übrigen sei auf die oben beschriebenen Vorteile des Systems verwiesen, die in analoger Weise auch für das erfindungsgemäße Verfahren gelten. Dies gilt entsprechend auch für die nachfolgend beschriebenen vorteilhaften Weiterbildungen des erfindungsgemäßen Verfahrens.

Günstigerweise wird der Mensch akustisch und/oder optisch abgeschirmt.

Vorzugsweise werden zum Abschirmen das Gesicht und/oder mindestens ein Auge des Menschen bedeckt und/oder abgedeckt.

Günstig ist es, wenn eine Lichtdurchlässigkeit während des Abschirmens abhängig von einem Schlafzustand des Menschen verändert wird.

Vorteilhaft ist es, wenn der Mensch und/oder sein Gehör aktiv und/oder passiv vor Lärm und/oder Geräuschen geschützt werden.

Vorzugsweise wird zur Durchführung des Verfahrens eines der oben beschriebenen Systeme verwendet.

Günstig ist es, wenn das System mindestens teilweise am Kopf des Menschen getragen wird. Auf diese Weise kann es direkt auf die zum Einschlafen anzusprechenden Sinne einwirken, als da sind das Sehen, das Hören und das Riechen.

Um die Gefahr der Übertragung von Krankheiten zu vermeiden, ist es günstig, wenn das System vor der Benutzung durch eine andere Person desinfiziert wird.

Günstigerweise wird ein Desinfektionszustand des Systems oder mindestens einer Einrichtung desselben bestimmt.

Vorteilhaft ist es, wenn der detektierte Desinfektionszustand angezeigt wird.

Vorzugsweise erfolgt das Entspannen optisch und/oder akustisch und/oder olfaktorisch.

Vorteilhaft ist es, wenn zum Entspannen des Menschen Bilder, Zeichen, Farben und/oder Filme wiedergegeben werden und/oder er mit einem als angenehm empfundenen Licht bestrahlt wird.

Günstig ist es, wenn zum Entspannen des Menschen Musik, Geräusche oder Töne und Tonfolgen wiedergegeben werden.

Vorzugsweise werden zum Wecken Musik, Geräusche und/oder Töne oder Tonfolgen und/oder Filme, Bilder und/oder Zeichen wiedergegeben und/oder Licht verwendet.

Vorteilhaft ist es, wenn zum Entspannen des Menschen Duftstoffe abgegeben werden.

Vorzugsweise werden die Duftstoffe dosiert abgegeben.

Zur Durchführung des Verfahrens ist es günstig, wenn ein Schlaf- und/oder Wachzustand des Menschen detektiert wird.

Besonders einfach lässt sich der Schlaf- und/oder Wachzustand ermitteln, wenn zu diesem Zweck Vitalfunktionen des Menschen optisch und/oder akustisch und/oder mittels Elektroden oder Sensoren detektiert werden.

Optional ist es günstig, wenn zum Ermitteln des Schlaf- und/oder Wachzustands die Atmung des Menschen, insbesondere die Atemfrequenz, gemessen und/oder überwacht wird.

Ferner kann es vorteilhaft sein, wenn zum Ermitteln des Schlaf- und/oder Wachzustands ein Blutdruck des Menschen gemessen und/oder überwacht wird.

Günstig ist es, wenn zum Ermitteln des Schlaf- und/oder Wachzustands eine Herzfrequenz des Menschen gemessen und/oder überwacht wird.

Gemäß einer bevorzugten Variante des Verfahrens ist es vorteilhaft, wenn zum Ermitteln des Schlaf- und/oder Wachzustands eine Augenbewegung des Menschen gemessen und/oder überwacht wird.

Vorzugsweise werden zum Ermitteln des Schlaf- und/oder Wachzustands Hirnströme des Menschen gemessen und/oder überwacht.

Vorteilhafterweise werden die gemessenen Istwerte mit Soll- oder Normwerten von Schlafzuständen verglichen und den Vergleichsergebnissen ein Istzustand zugeordnet.

Günstig ist es, wenn eine Schlafdauer des Kurzschlafes vorgegeben wird.

Vorteilhaft ist es, wenn zum Wecken die Abschirmung mindestens teilweise entfernt und/oder abgeschaltet wird.

Gemäß einer bevorzugten Variante des Verfahrens ist es günstig, wenn der Mensch, soweit dies die äußeren Umstände zulassen, nur während einer REM-Schlafphase geweckt wird.

Um einen möglichst erholsamen Schlaf des Menschen zu ermöglichen, ist es vorteilhaft, wenn der Mensch während der Durchführung des Verfahrens mit Frischluft versorgt wird.

Die nachfolgende Beschreibung bevorzugter Ausführungsformen der Erfindung dient im Zusammenhang mit der Zeichnung der näheren Erläuterung. Es zeigen:
- Figur 1:: eine schematische Darstellung eines erfindungsgemäßen Systems;
- Figur 2:: eine erste Ausführungsform eines Teils eines erfindungsgemäßen Systems;
- Figur 3:: eine schematische Darstellung eines Schnitts durch einen Teil einer akustischen Abschirmeinrichtung;
- Figur 4:: eine Schnittansicht ähnlich Figur 2 durch eine alternative Ausführungsform eines Teils einer akustischen Abschirmeinrichtung;
- Figur 5:: eine Ansicht ähnlich Figur 1 einer weiteren Ausführungsform eines Teils eines erfindungsgemäßen Systems;
- Figur 6:: eine teilweise Darstellung einer weiteren alternativen Ausführungsform eines erfindungsgemäßen Systems; und
- Figur 7:: ein Ablaufdiagramm einer Variante des erfindungsgemäßen Verfahrens.

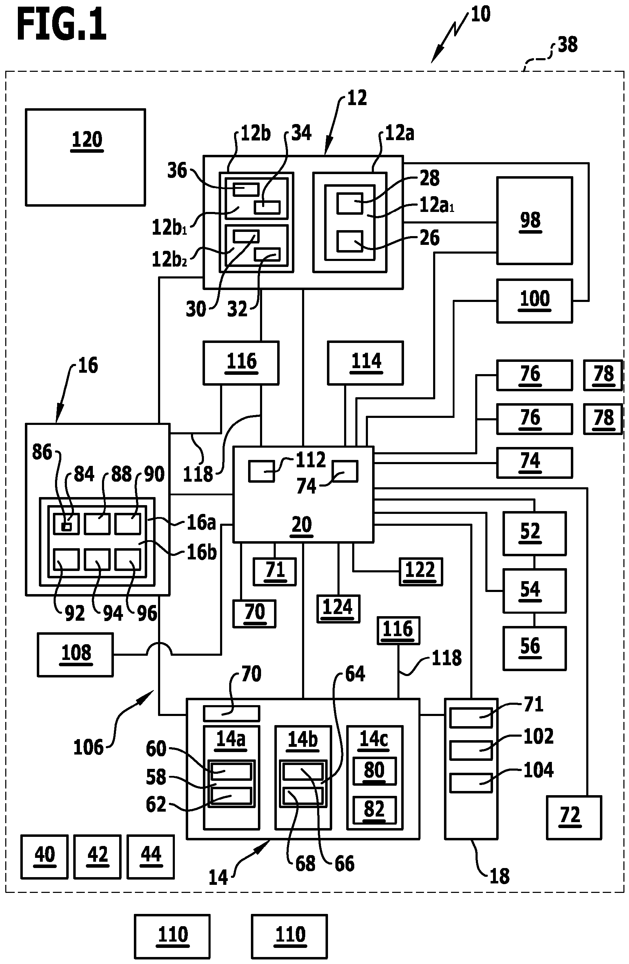

Der schematische Aufbau eines insgesamt mit dem Bezugszeichen 10 versehenen Systems zur Entspannung und/oder zur Unterstützung, Einleitung und/oder Überwachung eines Kurzschlafes eines Menschen 11 ist beispielhaft in Figur 1 dargestellt. Es umfasst eine Abschirmeinrichtung 12 zum mindestens teilweisen Abschirmen des Menschen 11 von seiner Umgebung 13, eine Entspannungseinrichtung 14 zum Entspannen und/oder Einleiten des Einschlafens des Menschen 11, eine Schlafdetektionseinrichtung 16 zum Feststellen, ob der Mensch 11 eingeschlafen ist, und eine Weckeinrichtung 18 zum Wecken des Menschen 11 nach einer vorgegebenen Schlafdauer. Die hier genannten Einrichtungen sind optional untereinander und/oder mit einer beispielsweise zentralen Steuer- und/oder Regelungseinrichtung 20 gekoppelt. Die Kopplung ist in Figur 1 in Form von Linienverbindungen dargestellt. Alle Verbindungen zwischen den Einrichtungen können in Form von Kabelverbindungen oder auch in Form von kabellosen Verbindungen, zum Beispiel mittels Funk, Infrarot und/oder Ultraschall, ausgebildet sein.

Die Abschirmeinrichtung 12 umfasst eine optische Abschirmeinrichtung 12a und eine akustische Abschirmeinrichtung 12b.

Die optische Abschirmeinrichtung 12a umfasst eine Gesichts- und/oder Augenabdeckungseinrichtung 12a₁ zum mindestens teilweisen Bedecken oder Abdecken des Gesichts 22 und/oder mindestens eines Auges 24 des Menschen 11. Sie ist vorzugsweise in Richtung auf das Gesicht 22 lichtundurchlässig. In Richtung vom Gesicht 22 des Menschen 11 weg ist sie vorzugsweise teilweise lichtdurchlässig. Des Weiteren umfasst die Gesicht- und/oder Augenabdeckungseinrichtung 12a₁ mindestens eine Augenklappe und/oder ein Visier 26 und/oder eine Mundabdeckung 28.

Die akustische Abschirmeinrichtung 12b umfasst eine aktive Lärm- und/oder Gehörschutzeinrichtung 12b₁ und/oder eine passive Lärm- und/oder Gehörschutzeinrichtung 12b₂. Die passive Lärm- und/oder Gehörschutzeinrichtung 12b₂ kann in Form eines Kapselgehörschutzes 30 oder in Form von Gehörschutzstöpseln 32 ausgebildet sein. Die aktive Lärm- und/oder Gehörschutzeinrichtung 12b₁ umfasst mindestens einen Ohrhörer 34 mit einer aktiven Umgebungsgeräuschminderungseinrichtung 36 zur aktiven Reduzierung von permanent und/oder temporär auftretenden Umgebungsgeräuschen.

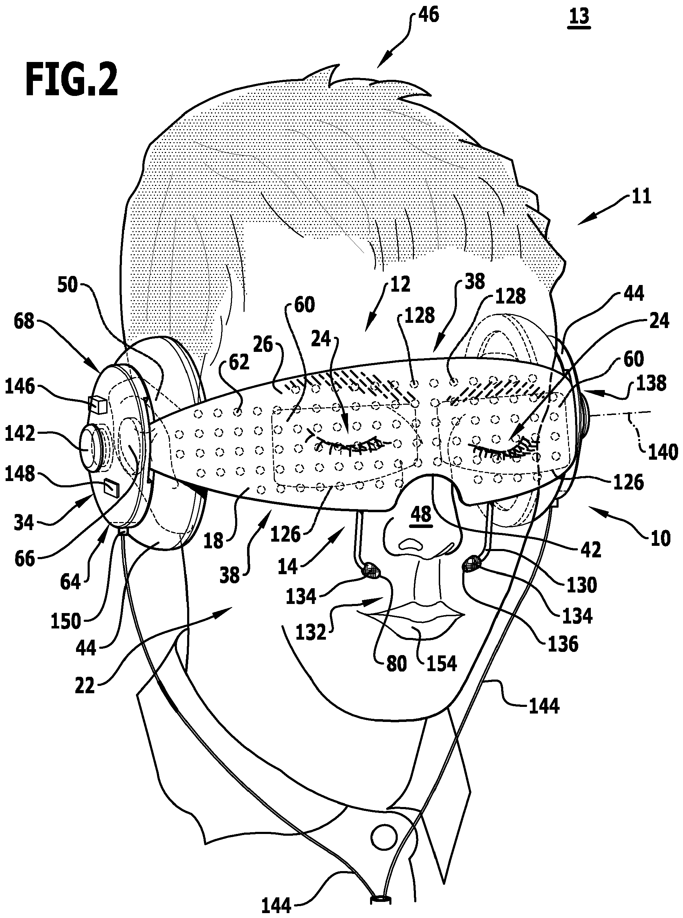

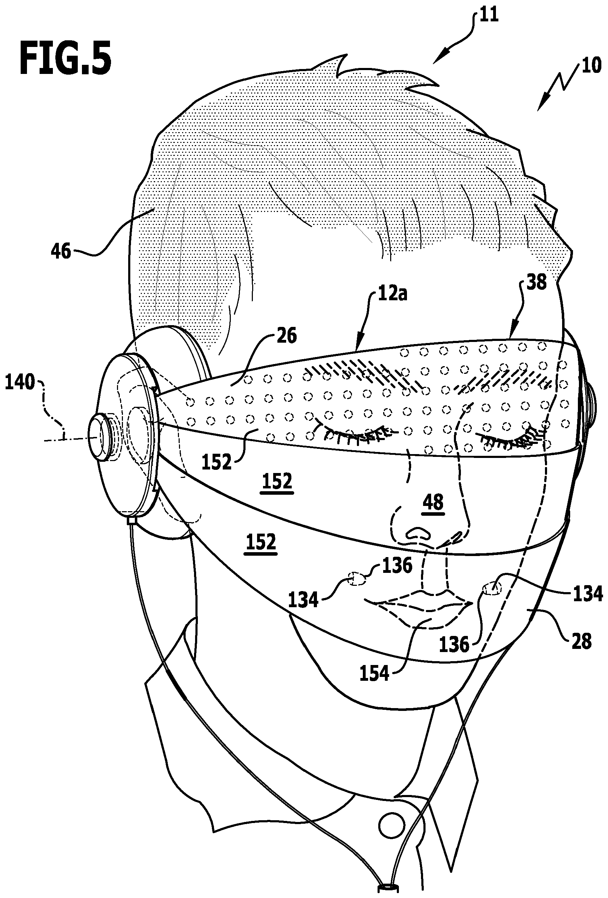

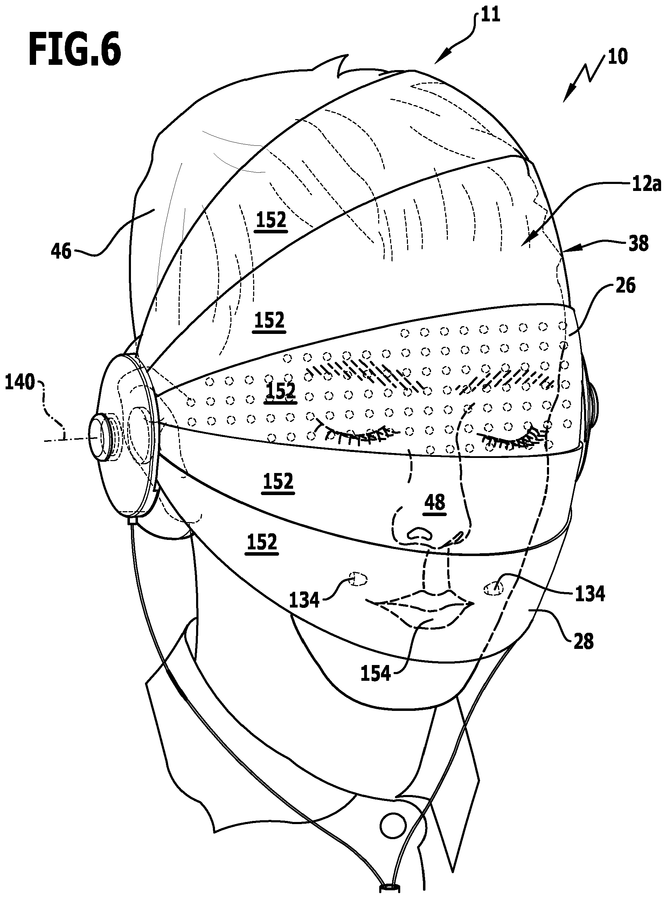

Das System 10 umfasst ferner einen Träger 38, der in Figur 1 schematisch als gestrichelter Kasten dargestellt ist. Am Träger 38 können wahlweise die Abschirmeinrichtung 12 und/oder die Entspannungseinrichtung 14 und/oder die Schlafdetektionseinrichtung 16 und/oder die Weckeinrichtung 18 mindestens teilweise gehalten sein. Ferner können am Träger 38 auch alle weiteren Einrichtungen des Systems 10 angeordnet sein, sei es die bereits oben beschriebenen Einrichtungen oder die nachfolgend noch näher beschriebenen Einrichtungen. Der Träger 38 kann ein Kopf- 40 und/oder ein Nasen- 42 und/oder ein Ohrenauflageteil 44 umfassen zum An- und/oder Auflegen des Trägers 38 auf den Kopf 46 und/oder die Nase 48 und/oder ein Ohr 50 des Menschen 11. Der Träger 38 kann, wie beispielsweise in den Figuren 5 und 6 dargestellt, in Form eines Helmes oder einer Haube ausgebildet sein, alternativ auch in Form eines Hutes, eines Kopftuches oder eines Schals. Denkbar wäre es auch, den Träger 38, wie in Figur 2 beispielhaft dargestellt, brillenförmig auszubilden.

Das System 10 umfasst ferner eine Desinfektionseinrichtung 52 zum Desinfizieren mindestens einer der Einrichtungen des Systems 10. Es umfasst des Weiteren eine Desinfektionszustandsdetektionseinrichtung 54 zum Detektieren eines Desinfektionszustands des Systems 10 oder mindestens einer Einrichtung desselben. Zum Anzeigen des detektierten Desinfektionszustands dient eine Desinfektionszustandsanzeige 56.

Die Entspannungseinrichtung 14 ist in Form einer optischen Entspannungseinrichtung 14a und/oder einer akustischen Entspannungseinrichtung 14b und/oder einer olfaktorischen Entspannungseinrichtung 14c ausgebildet.

Die optische Entspannungseinrichtung 14a umfasst mindestens eine optische Wiedergabeeinrichtung 58 zum Wiedergeben und Anzeigen von Bildern, Zeichen, Farben und/oder Filmen. Sie umfasst mindestens einen Bildschirm 60 und/oder mindestens ein Leuchtelement 62, welches beispielsweise in Form einer Leuchtdiode, insbesondere einer einfarbigen Diode oder Multicolorleuchtdiode ausgebildet ist.

Die akustische Entspannungseinrichtung 14b umfasst mindestens eine akustische Wiedergabeeinrichtung 64 zum Wiedergeben von Musik, Geräuschen oder Tönen und Tonfolgen. Diese wiederum umfasst mindestens einen Lautsprecher 66 oder mindestens einen Kopf- oder Ohrhörer 68. Des Weiteren können eine oder mehrere Schnittstelle 70 oder 71 zum Verbinden der Entspannungseinrichtung 14 und/oder der Weckeinrichtung 18 mit einer Abspieleinrichtung 72 zum Abspielen von Ton- und/oder Bilddaten vorgesehen sein. Die Schnittstellen 70, 71 können insbesondere auch einen Teil des Systems 10 bilden und mit der Steuer- und/oder Regelungseinrichtung 20 verbunden sein. Des Weiteren können die Schnittstellen 70, 71 insbesondere in Form einer USB- oder Firewire-Schnittstelle ausgebildet sein oder eine Schnittstelle für eine Bluetooth- oder kabelfreie Verbindung bilden.

Das System 10 umfasst ferner eine oder mehrere Speichereinrichtungen 74 zum Speichern von Ton- und/oder Bilddaten und/oder Computerprogrammen. Zum Lesen von Ton- und/oder Bilddaten von Datenträgern 78 ist mindestens eine Datenträgerleseeinheit 76 vorgesehen. Die Datenträgerleseeinheit 76 kann in Form eines Chipkartenlesers, eines Speicherkartenlesers, eines CD-Laufwerks, eines DVD-Laufwerks oder eines Video- und/oder Audiokassettenlaufwerks ausgebildet sein.

Die olfaktorische Entspannungseinrichtung 14c umfasst eine Duftabgabeeinrichtung 80 zum Abgeben von Duftstoffen, beispielsweise ätherischen Ölen. Die olfaktorische Entspannungseinrichtung 14c umfasst ferner eine mit der Duftabgabeeinrichtung 80 gekoppelte Dosiereinrichtung 82 zum individuellen Dosieren einer Duftstoffabgabemenge.

Die Schlafdetektionseinrichtung 16 umfasst ferner eine Schlafzustandsdetektionseinrichtung 16a zum Detektieren eines Schlaf- und/oder Wachzustands des Menschen 11. Diese wiederum umfasst eine optische und/oder akustische Detektionseinrichtung 16b zum Detektieren von Vitalfunktionen des Menschen 11. Eine Vitalfunktion des Menschen 11 ist beispielsweise seine Atmung. Die Schlafzustandsdetektionseinrichtung 16a umfasst daher eine Atmungsüberwachungseinrichtung 84 zum Messen und/oder Überwachen einer Atemfrequenz des Menschen. Die Atmungsüberwachungseinrichtung 84 umfasst vorzugsweise ein Mikrophon 86.

Des Weiteren umfasst die Schlafzustandsdetektionseinrichtung 16a eine Blutdrucküberwachungseinrichtung 88 zum Messen und/oder Überwachen eines Blutdrucks des Menschen 11.

Ferner umfasst die Schlafzustandsdetektionseinrichtung 16a eine Herzfrequenzüberwachungseinrichtung 90 zum Messen und/oder Überwachen einer Herzfrequenz des Menschen 11. Die Schlafzustandsdetektionseinrichtung 16a umfasst des Weiteren eine Augenbewegungsüberwachungseinrichtung 92 zum Messen und/oder Überwachen einer Augenbewegung des Menschen 11. Des Weiteren ist auch eine Hirnstromüberwachungseinrichtung 94 vorgesehen zum Messen und/oder Überwachen von Hirnströmen des Menschen 11. Die Schlafzustandsdetektionseinrichtung 16a umfasst ferner eine Vergleichs- und Zuordnungseinrichtung 96 zum Vergleichen von mit der Schlafzustandsdetektionseinrichtung 16a gemessenen Istwerten mit Sollwerten von Schlafzuständen und zum Zuordnen eines Istschlafzustandes in Abhängigkeit eines Vergleichsergebnisses der Ist- und Sollwerte.

Des Weiteren umfasst das System 10 eine Schlafdauervorgabeeinrichtung 98 zum Vorgeben einer Schlafdauer des Kurzschlafes. Mit einer Eingabeeinheit 100 kann beispielsweise eine Schlafdauer in einem Bereich von 5 Minuten bis 30 Minuten vorzugsweise bis zu 60 Minuten eingegeben werden, entweder direkt oder durch die mit der Eingabeeinheit 100 verbundene Schlafdauervorgabeeinrichtung 98 oder indirekt über die Steuer- und/oder Regelungseinrichtung 20.

Die Weckeinrichtung 18 ist derart ausgebildet, dass sie nach Ablauf der durch die Schlafdauervorgabeeinrichtung 98 vorgegebenen Schlafdauer aktivierbar ist oder bevor der Mensch 11 in den Tiefschlaf fällt, beispielsweise durch Ansteuerung über die Steuer- und/oder Regelungseinheit 20. Die Weckeinrichtung 18 umfasst mindestens eine optische Wiedergabeeinrichtung 102 oder eine akustische Wiedergabeeinrichtung 104. Die Weckeinrichtung 18 kann optional ausgebildet sein zum mindestens teilweisen Entfernen und/oder Abschalten der Abschirmeinrichtung 12.

Wie bereits dargelegt, dient die Steuer- und/oder Regelungseinrichtung 20 zum Steuern und/oder Regeln mindestens einer der Einrichtungen des Systems 10. Zu diesem Zweck können die Einrichtungen des Systems 10 durch entsprechende Verbindungen 106, beispielsweise kabelgebundene oder kabellose, untereinander oder aber auch zusätzlich über die Steuer- und/oder Regelungseinrichtung 20 miteinander gekoppelt sein. Falls eine Funkverbindung 106 vorgesehen ist, kann diese vorzugsweise in Form einer Bluetooth-Verbindung ausgebildet sein.

Des Weiteren umfasst das System 10 eine Funkverbindungseinrichtung 108 zum drahtlosen Verbinden einzelner Einrichtungen des Systems 10 untereinander und/oder mit externen Geräten 110.

Die Steuer- und/oder Regelungseinrichtung 20 umfasst mindestens eine Rechen- und/oder Datenverarbeitungseinheit 112 zum Verrechnen und/oder Verarbeiten von mit den einzelnen Einrichtungen des Systems 10 gemessenen oder erfassten Daten und/oder vorgegebenen Daten oder Sollwerten. Die Steuer- und/oder Regelungseinrichtung 20 ist insbesondere ausgebildet zum Ansteuern der Abschirmeinrichtung 12 und der Entspannungseinrichtung 14. Ferner kann mit der Steuer- und/oder Regelungseinrichtung 20 auch die Duftabgabeeinrichtung 80 angesteuert werden. Des Weiteren ist auch die Ansteuerung der Schlafdauervorgabeeinrichtung 98 vorgesehen. Darüber hinaus ist auch die Weckeinrichtung 18 von der Steuer- und/oder Regelungseinrichtung 20 in Abhängigkeit der vorgegebenen Schlafdauer und des mit der Schlafzustandsdetektionseinrichtung 16a detektierten Schlafzustands ansteuerbar.

Die Weckeinrichtung 18 ist vorzugsweise so ausgebildet, dass sie nur während einer REM-Schlafphase des Menschen 11 aktivierbar ist.

Zur Versorgung des Menschen 11 mit Frischluft dient eine Belüftungseinrichtung 114.

Um das System 10 an beliebigen Orten einsetzen zu können, ist eine netzunabhängige Energieversorgungseinheit 116, beispielsweise eine Batterie oder ein Akku, für mindestens eine Einrichtung des Systems 10 vorgesehen. In Figur 1 sind beispielhaft nur Verbindungs- oder Versorgungsleitungen 118 zwischen der Energieversorgungseinheit 116 sowie der Abschirmeinrichtung 12 und der Schlafdetektionseinrichtung 16 sowie der Steuer- und/oder Regelungseinrichtung 20 dargestellt. Derartige Versorgungsleitungen 118 sind auch zu allen anderen Einrichtungen und Geräten des Systems 10 denkbar. Optional können auch weitere Energieversorgungseinheiten 116 vorgesehen sein, die beispielsweise nur einer einzigen Einrichtung des Systems 10 zugeordnet und mit dieser über eine Versorgungsleitung 118 verbunden sind, wie dies beispielhaft in Figur 1 zur Versorgung der Entspannungseinrichtung 14 mit Energie dargestellt ist.

In Figur 1 ist ferner schematisch eine Transporteinrichtung 120 dargestellt, die derart ausgebildet ist, dass sie alle Teile des Systems 10 oder mindestens eine Einrichtung desselben für Transportzwecke aufnehmen können. Die Transporteinrichtung 120 kann beispielsweise in Form eines Beutels, einer Tasche oder eines Koffers ausgebildet sein. Um das System 10 beispielsweise mit einem Mobiltelefon und/oder dem Internet verbinden zu können, ist eine Schnittstelle 122 vorgesehen. Diese ist beispielsweise mit der Steuer- und/oder Regelungseinheit 20 gekoppelt, so dass insbesondere eingehende Telefonanrufe mit der Steuer- und/oder Regelungseinrichtung 20 verarbeitet werden können, zum Beispiel zum Aktivieren der Weckeinrichtung 18.

Eine Eingabeeinrichtung 124 zum Eingeben von Daten und/oder Vorgabewerten für mindestens eine Einrichtung und/oder Vorrichtung des Systems 10 und/oder mit dem System 10 verbundener oder gekoppelter Vorrichtungen oder Geräte 110 ist beispielsweise mit der Steuer- und/oder Regelungseinrichtung 20 verbunden. Die Eingabeeinrichtung 124 kann insbesondere in Form einer Fernbedienung ausgebildet sein, die zudem auch die Steuer- und/oder Regelungseinrichtung 20 umfassen kann. Dies ermöglicht es, das System und dessen Einrichtungen bequem und ohne Kabelgewirr zu bedienen, insbesondere dann, wenn der Mensch 11 den Träger 38 auf dem Kopf 46 trägt.

Unter Bezugnahme auf Figur 2 wird nachfolgend eine erste Ausführungsform des Systems 10 beispielhaft erläutert. Es umfasst einen brillenförmigen Träger, welcher undurchsichtig ist und auf seiner Innenseite wahlweise kleine, jeweils vor den Augen 24 angeordnete, Bildschirme 60 bildende Displays 126 oder eine Vielzahl von Leuchtelementen 62 in Form von in einem regelmäßigen Raster angeordneten LEDs 128 aufweist. Die Displays 126 und die LEDs 128 bilden einen Teil der optischen Entspannungseinrichtung 14a des Systems 10. Der visierartig ausgebildete einstückige Träger 18 weist eine halbkreisförmige Aussparung auf, die ein Nasenauflageteil 42 bildet. Benachbart des Nasenauflageteils 42 stehen im Wesentlichen C-förmig geschwungene Bügel 130 ab, deren freie Enden im Mund- und/oder Nasenbereich 132 enden. Die Bügel 130 tragen an ihren freien Enden jeweils ein Mikrophon 134, mit welchem beispielsweise Atemstöße des Menschen 11 gemessen und detektiert werden können. Die Bügel 130 sind vorzugsweise hohl und bilden einen Kanal für Duftstoffe, die aus den freien Enden der Bügel 130 im Bereich der Mikrophone 134 aus Auslässen 136 austreten können. Die olfaktorische Entspannungseinrichtung 14c umfasst zu diesem Zweck einen nicht dargestellten Duftstoffspeicher, welcher mit der Duftstoffabgabeeinrichtung 80 gekoppelt ist, wobei eine Dosierung der Duftstoffabgabemenge mit der Dosiereinrichtung 82 realisiert wird.

Die LEDs 128 können entweder angesteuert werden zum Erzeugen eines warmen beruhigenden Lichts im Augenbereich oder zur Erzeugung eines hellen Wecklichts.

Die Mikrophone 134 können auch mit einem Mobiltelefon gekoppelt sein, so dass der Mensch 11, mittels des Systems 10 auch mit anderen Menschen kommunizieren kann.

Ohrhörer 34 bilden sowohl einen Teil der akustischen Abschirmeinrichtung 12b als auch der akustischen Entspannungseinrichtung 14b. Sie sind in einer gekapselten Ausführung vorgesehen und umfassen ein Scharniergelenk 138, welches ein Verschwenken des Visiers 26 relativ zu den Ohrhörern 34 um eine Schwenkachse 140 gestattet. Ein außen am Ohrhörer 34 angeordnetes Mikrophon 142 dient zur Messung von Umgebungslärm und/oder -geräuschen und bildet einen Teil der aktiven akustischen Abschirmeinrichtung 12b₁. Innen, dem Ohr 50 des Menschen 11 zugewandt, ist am Ohrhörer 34 ein Lautsprecher 66 angeordnet. Über Steuerleitungen 144 sind die Ohrhörer 34 und die an diesen angeordneten Einrichtungen mit der Steuer- und/oder Regelungseinrichtung 20 verbunden. Am oder im Ohrhörer 34 kann eine Elektronik 146 angeordnet sein, welche insbesondere zur Steuerung und/oder Regelung des Mikrophons 142 sowie der aktiven Umgebungslärmunterdrückung dient. Ferner kann sie für die Ansteuerung des Lautsprechers 66 zur Wiedergabe von Musik oder beruhigende Klangfolgen dienen sowie zur Auslösung und Wiedergabe eines Weckalarms. Ferner kann die Elektronik 146 auch die Mikrophone 134 ansteuern sowie die Displays 126 beziehungsweise die Leuchtdioden 128. Ebenfalls ansteuerbar über die Elektronik 146 ist die Duftdosierung. Der Lautsprecher 66 kann auch einen Teil eines Mobiltelefons bilden. Zur Energieversorgung kann eine Batterie 148 am Ohrhörer 34 angeordnet sein. Die Elektronik 146 kann ferner auch einen Verbindungssender zur Hauptelektronik des Systems 10 in Form der Steuer- und/oder Regelungseinheit 20 umfassen. Die Steuerleitungen 144 sind über an den Ohrhörern 34 vorgesehene Buchsen mit den Ohrhörern 34 lösbar verbindbar.

Alternativ kann mit der Elektronik 146 auch die getrennt vom Träger 38 angeordnete und mit diesem optional nur über eine Funkverbindung verbundene Steuer- und/oder Regelungseinrichtung 20 die oben beschriebenen Funktionen der Elektronik 146 übernehmen.

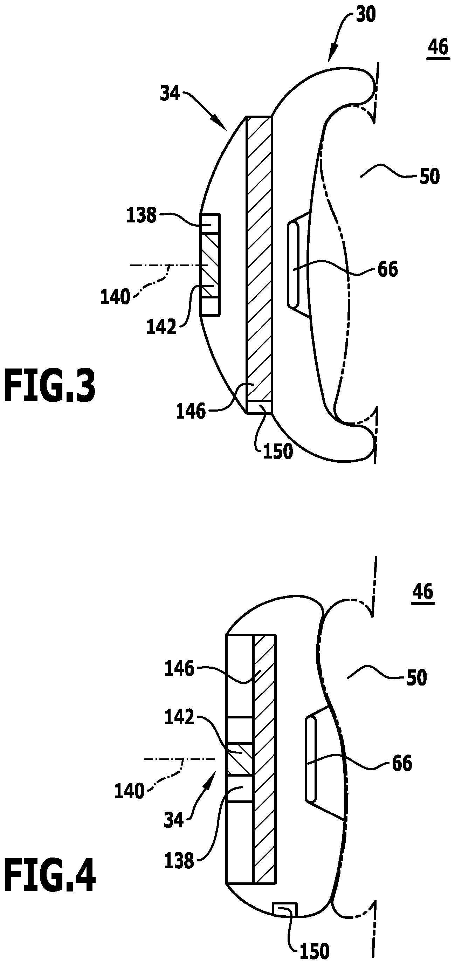

Der Ohrhörer 34 kann in Form eines sogenannten geschlossenen Ohrhörersystems ausgebildet sein, wie es in den Figuren 2, 3 und 5 beispielhaft dargestellt ist. Der Ohrhörer 34 bildet somit gleichzeitig auch einen Kapselgehörschutz 30, der das Ohr 50 des Menschen 11 vollständig umschließt. Alternativ kann der Ohrhörer 34, wie in den Figuren 4 und 6 beispielhaft dargestellt, auch in offener Bauweise ausgebildet sein, so dass er nur auf dem Ohr 50 aufliegt. Die Funktionen sowohl des Ohrhörers 34 in offener als auch geschlossener Bauweise stimmen entsprechend überein.

In den Figuren 5 und 6 sind alternative Ausführungsformen des Systems 10 dargestellt. Der Träger 38 umfasst bei dem in Figur 5 dargestellten Ausführungsbeispiel drei im Wesentlichen identische, im Wesentlichen halbkreisförmig gebogene, streifenförmige Segmente 152, wobei eines im Wesentlichen identisch mit dem in Figur 2 dargestellten einteiligen Träger 38 ausgebildet ist. Die drei Segmente 152 sind mittels des Scharniergelenks 138 um die gemeinsame Schwenkachse 140 verschwenkbar gelagert. Die drei Segmente gestatten es, nicht nur den Augenbereich des Gesichts 22 zu bedecken, sondern auch die Nase 48 und den Mund 154. Dadurch kann eine noch bessere Abschirmung des Menschen 11 zu einer Umgebung hin erreicht werden. Die Mikrophone 134 können direkt am untersten der drei Segmente 152 angeordnet sein. Gleiches gilt auch für die Auslässe 136 der Duftstoffabgabeeinrichtung 80. Die Segmente 152 können zu Belüftungszwecken auch perforiert sein. Vorzugsweise sind sie aus Metall oder Kunststoff hergestellt.

Um eine bessere Abkapselung und räumliche Abtrennung des Menschen 11 von seiner Umgebung zu erreichen, können zusätzlich oberhalb des die Augen 24 abdeckenden Segments 152 zwei weitere um die Schwenkachse 140 verschwenkbare Segmente 152 vorgesehen sein. Die relativ zueinander angeordneten Segmente 152, wie die in den Figuren 5 und 6 dargestellten Ausführungsbeispiele zeigen, ermöglichen es, die optische Abschirmeinrichtung 12a zu Transportzwecken zusammenzuschwenken, wobei dann die Segmente 152 in einer Transportstellung übereinander liegen und so nur eine minimale Fläche überdecken, die in etwa der eines einzelnen Segments 152 entspricht.

Die in den Figuren beispielhaft dargestellten visierartigen oder in Form einer Einscheibenbrille ausgestalteten Träger 38 können optional auch durch alle anderen möglichen und oben erwähnten Träger 38 ersetzt werden. Die beschriebenen Einrichtungen des Systems 10 können an den jeweiligen Trägern 38 in geeigneter Weise angeordnet und angesteuert werden.

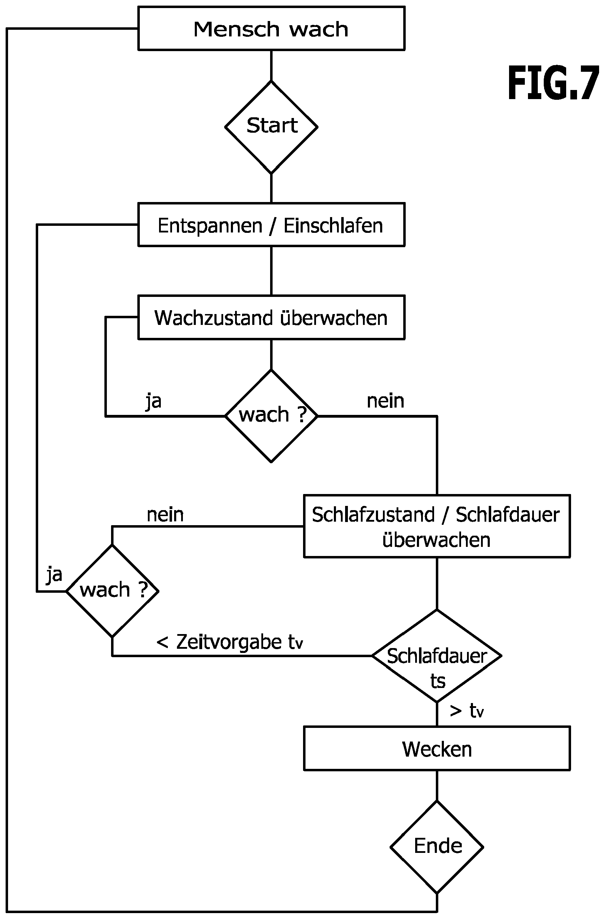

Mit Bezug zur Figur 7 wird ein Beispiel des erfindungsgemäßen Verfahrens anhand des dargestellten Ablaufdiagramms kurz erläutert.

Vor einem Einsatz des Systems 10 ist der Mensch 11 vorzugsweise wach. Er startet das System 10, das ihn durch entsprechende optische und/oder akustische Reize zum Entspannen beziehungsweise zum Einschlafen anregt beziehungsweise einlädt, durch Starten des Programms und Eingabe des gewünschten Weckverhaltens. Dabei wird sein Wachzustand überwacht. Diese Überwachung wird solange fortgesetzt, bis der Nutzer nicht mehr wach ist. Es werden dann sein Schlafzustand und die vorgegebene Schlafdauer überwacht. Ist die Schlafdauer t_{S} kleiner als die vorgegebene Schlafdauer t_{V}, wird überprüft, ob der Mensch 11 wach ist oder noch schläft. Schläft er noch, werden sein Schlafzustand und die Schlafdauer t_{S} weiter überwacht, und zwar solange, bis die Schlafdauer t_{S} gleich oder größer der vorgegebenen Schlafdauer tᵥ ist. Dann wird der Mensch 11 geweckt und das Verfahren ist beendet. Sollte der Mensch 11 vor Ablauf der vorgegebenen Schlafdauer t_{V} aufwachen, wird er wiederum durch die eingestellten optischen und/oder akustischen Reize zum Entspannen beziehungsweise Einschlafen angeregt. Das Wecken kann mittels akustischer und/oder optischer Reize mittels der Weckeinrichtung 18 erreicht werden.

## Patentansprüche

1. System (10) zur Unterstützung, Einleitung und Überwachung eines Kurzschlafs eines Menschen (11), mit einer Abschirmeinrichtung (12) zum mindestens teilweisen Abschirmen des Menschen (11) von seiner Umgebung (13) und mit einer Entspannungseinrichtung (14) zum Entspannen und Einleiten des Einschlafens des Menschen (11), wobei es ausgebildet ist zur Überwachung eines Kurzschlafs eines Menschen (11) und eine Schlafdetektionseinrichtung (16) umfasst zum Feststellen, ob der Mensch (11) eingeschlafen ist, **dadurch gekennzeichnet, dass** es eine Weckeinrichtung (18) zum Wecken des Menschen (11) vor Eintritt einer Tiefschlafphase umfasst.

2. System nach Anspruch 1, **dadurch gekennzeichnet, dass** die Abschirmeinrichtung (12) eine optische und/oder akustische Abschirmeinrichtung (12a, 12b) umfasst.

3. System nach Anspruch 2, **dadurch gekennzeichnet, dass** eine Lichtdurchlässigkeit der optischen Abschirmeinrichtung (12a) veränderbar ist.

4. System nach einem der voranstehenden Ansprüche, **gekennzeichnet durch** eine Desinfektionseinrichtung (52) zum Desinfizieren mindestens einer Einrichtung des Systems (10).

5. System nach einem der voranstehenden Ansprüche, **gekennzeichnet durch** eine Desinfektionszustandsdetektionseinrichtung (54) zum Detektieren eines Desinfektionszustands des Systems (10) oder mindestens einer Einrichtung desselben.

6. System nach einem der voranstehenden Ansprüche, **dadurch gekennzeichnet, dass** die Entspannungseinrichtung (14) in Form einer optischen und/oder akustischen und/oder olfaktorischen Entspannungseinrichtung (14a, 14b, 14c) ausgebildet ist.

7. System nach einem der voranstehenden Ansprüche, **dadurch gekennzeichnet, dass** die Schlafdetektionseinrichtung (16) eine Schlafzustandsdetektionseinrichtung (16a) zum Detektieren eines Schlaf- und/oder Wachzustands des Menschen (11) umfasst.

8. System nach Anspruch 7, **dadurch gekennzeichnet, dass** die Schlafzustandsdetektionseinrichtung (16a) eine optische und/oder akustische Detektionseinrichtung (16b) zum Detektieren von Vitalfunktionen des Menschen (11) umfasst.

9. System nach Anspruch 7 oder 8, **dadurch gekennzeichnet, dass** die Schlafzustandsdetektionseinrichtung (16a) eine Vergleichs- und Zuordnungseinrichtung (96) umfasst zum Vergleichen von mit der Schlafzustandsdetektionseinrichtung (16a) gemessener Istwerte mit Sollwerten von Schlafzuständen und zum Zuordnen eines Ist-Schlafzustands in Abhängigkeit eines Vergleichsergebnisses der Ist- und Sollwerte.

10. System nach einem der voranstehenden Ansprüche, **gekennzeichnet durch** eine Schlafdauervorgabeeinrichtung (98) zum Vorgeben einer Schlafdauer des Kurzschlafes.

11. System nach einem der voranstehenden Ansprüche, **gekennzeichnet durch** mindestens eine Steuer- und/oder Regelungseinrichtung (20) zum Steuern und/oder Regeln mindestens einer der Einrichtungen des Systems (10).

12. System nach einem der voranstehenden Ansprüche, **gekennzeichnet durch** eine kabellose Verbindungseinrichtung (108) zum drahtlosen Verbinden einzelner Einrichtungen des Systems (10) untereinander und/oder mit externen Geräten (110).

13. System nach einem der voranstehenden Ansprüche, **dadurch gekennzeichnet, dass** die Weckeinrichtung (18) nur während einer REM-Schlafphase des Menschen (11) aktivierbar ist.

14. System nach einem der voranstehenden Ansprüche, **gekennzeichnet durch** eine Eingabeeinrichtung (124) zum Eingeben von Daten und/oder Vorgabewerten für mindestens eine Einrichtung und/oder Vorrichtung des Systems (10) und/oder mit dem System (10) verbundener oder gekoppelter Vorrichtungen oder Geräte (110).

15. Verfahren zur Unterstützung, Einleitung und Überwachung eines Kurzschlafs eines Menschen, bei welchem der Mensch mindestens teilweise von seiner Umgebung abgeschirmt und eine Entspannung und ein Einleiten des Einschlafens des Menschen durchgeführt wird, wobei detektiert wird, ob der Mensch eingeschlafen ist und ein Kurzschlaf des Menschen überwacht wird, **dadurch gekennzeichnet, dass** der Mensch vor Eintritt einer Tiefschlafphase wieder geweckt wird.

## Claims

1. System (10) for supporting, inducing and monitoring a nap of a person (11), comprising a shielding device (12) for at least partially shielding the person (11) from his environment (13), and comprising a relaxation device (14) for relaxing and inducing the person (11) to fall asleep, the system being configured to monitor a nap of a person (11) and comprising a sleep detection device (16) for determining whether the person (11) has fallen asleep, **characterized in that** the system comprises a wake-up device (18) for waking the person (11) before entry into a deep sleep phase.

2. System in accordance with claim 1, **characterized in that** the shielding device (12) comprises an optical and/or acoustic shielding device (12a, 12b).

3. System in accordance with claim 2, **characterized in that** a translucence of the optical shielding device (12a) is alterable.

4. System in accordance with any one of the preceding claims, **characterized by** a disinfection device (52) for disinfecting at least one device of the system (10).

5. System in accordance with any one of the preceding claims, **characterized by** a disinfection state detection device (54) for detecting a disinfection state of the system (10) or at least one device thereof.

6. System in accordance with any one of the preceding claims, **characterized in that** the relaxation device (14) is configured in the form of an optical and/or acoustic and/or olfactory relaxation device (14a, 14b, 14c).

7. System in accordance with any one of the preceding claims, **characterized in that** the sleep detection device (16) comprises a sleep state detection device (16a) for detecting a sleep state and/or awake state of the person (11).

8. System in accordance with claim 7, **characterized in that** the sleep state detection device (16a) comprises an optical and/or acoustic detection device (16b) for detecting vital functions of the person (11).

9. System in accordance with claim 7 or 8, **characterized in that** the sleep state detection device (16a) comprises a comparison and allocation device (96) for comparing actual values measured with the sleep state detection device (16a) with set values of sleep states and for allocating an actual sleep state in dependence upon a result of comparison of the actual and set values.

10. System in accordance with any one of the preceding claims, **characterized by** a sleep duration specifying device (98) for specifying a sleep duration of the nap.

11. System in accordance with any one of the preceding claims, **characterized by** at least one control and/or regulating device (20) for controlling and/or regulating at least one of the devices of the system (10).

12. System in accordance with any one of the preceding claims, **characterized by** a cable-free connection device (108) for wirelessly connecting individual devices of the system (10) to one another and/or to external apparatuses (110).

13. System in accordance with any one of the preceding claims, **characterized in that** the wake-up device (18) is only activatable during a REM sleep phase of the person (11).

14. System in accordance with any one of the preceding claims, **characterized by** an input device (124) for inputting data and/or specified values for at least one device and/or appliance of the system (10) and/or appliances or apparatuses (110) connected or coupled to the system (10).

15. Method for supporting, inducing and monitoring a nap of a person, wherein the person is at least partially shielded from his environment, and a relaxing and an inducing of the person to fall asleep are carried out, and wherein it is detected whether the person has fallen asleep, and a nap of the person is monitored, **characterized in that** the person is woken again before entry into a deep sleep phase.

## Revendications

1. Système (10) pour favoriser ou stimuler, initier et surveiller un sommeil de courte durée d'un être humain ou d'une personne (11), comprenant un dispositif d'isolement (12) destiné à l'isolement au moins partiel de la personne (11) de son environnement (13), et comprenant un dispositif de détente (14) pour détendre et initier l'endormissement de la personne (11), le système étant conçu pour surveiller un sommeil de courte durée d'une personne (11) et comprenant un dispositif de détection de sommeil (16) pour détecter si la personne (11) est endormie, **caractérisé en ce qu'**il comprend un dispositif de réveil (18) destiné à réveiller la personne (11) avant l'apparition d'une phase de sommeil profond.

2. Système selon la revendication 1, **caractérisé en ce que** le dispositif d'isolement (12) comprend un dispositif d'isolement optique et/ou acoustique (12a, 12b) .

3. Système selon la revendication 2, **caractérisé en ce qu'**il est possible de faire varier une transparence à la lumière du dispositif d'isolement optique (12a).

4. Système selon l'une des revendications précédentes, **caractérisé par** un dispositif de désinfection (52) pour désinfecter au moins un dispositif du système (10).

5. Système selon l'une des revendications précédentes, **caractérisé par** un dispositif de détection d'état de désinfection (54) destiné à détecter un état de désinfection du système (10) ou d'au moins un dispositif de celui-ci.

6. Système selon l'une des revendications précédentes, **caractérisé en ce que** le dispositif de détente (14) est réalisé sous la forme d'un dispositif de détente optique et/ou acoustique et/ou olfactif (14a, 14b, 14c).

7. Système selon l'une des revendications précédentes, **caractérisé en ce que** le dispositif de détection de sommeil (16) comprend un dispositif de détection d'état de sommeil (16a) destiné à détecter un état de sommeil et/ou de réveil de la personne (11).

8. Système selon la revendication 7, **caractérisé en ce que** le dispositif de détection d'état de sommeil (16a) comprend un dispositif de détection optique et/ou acoustique (16b) pour détecter des fonctions vitales de la personne (11).

9. Système selon la revendication 7 ou la revendication 8, **caractérisé en ce que** le dispositif de détection d'état de sommeil (16a) comprend un dispositif de comparaison et d'affectation (96) pour comparer des valeurs réelles instantanées mesurées avec le dispositif de détection d'état de sommeil (16a) à des valeurs de consigne d'état de sommeil, et affecter un état de sommeil réel instantané en fonction d'un résultat de comparaison des valeurs réelles instantanées et des valeurs de consigne.

10. Système selon l'une des revendications précédentes, **caractérisé par** un dispositif de prescription de durée de sommeil (98) destiné à prescrire une durée de sommeil dudit sommeil de courte durée.

11. Système selon l'une des revendications précédentes, **caractérisé par** au moins un dispositif de commande et/ou de régulation (20) destiné à commander et/ou réguler au moins l'un des dispositifs du système (10).

12. Système selon l'une des revendications précédentes, **caractérisé par** un dispositif de liaison sans câble (108) pour assurer une liaison sans fil entre différents dispositifs du système (10) et/ou avec des appareils extérieurs (110).

13. Système selon l'une des revendications précédentes, **caractérisé en ce que** le dispositif de réveil (18) ne peut être activé que lors d'une phase de sommeil REM (sommeil paradoxal) de la personne (11).

14. Système selon l'une des revendications précédentes, **caractérisé par** un dispositif de saisie (124) pour la saisie des données et/ou des valeurs de consigne pour au moins un dispositif et/ou ustensile du système (10) et/ou des ustensiles ou appareils (110) reliés ou couplés au système (10).

15. Procédé pour favoriser ou stimuler, initier et surveiller un sommeil de courte durée d'un être humain ou d'une personne, d'après lequel la personne est au moins partiellement isolée de son environnement, et d'après lequel on assure une détente et l'initiation de l'endormissement de la personne, et l'on détecte si la personne est endormie tout en assurant la surveillance d'un sommeil de courte durée de la personne, **caractérisé en ce que** l'on procède au réveil de la personne avant l'apparition d'une phase de sommeil profond.
